Europäisches Patentamt

European Patent Office

Office européen des brevets

(19)

(11) Veröffentlichungsnummer: **0 250 786 B1**

(12) **EUROPÄISCHE PATENTSCHRIFT**

(45) Veröffentlichungstag der Patentschrift: **30.09.92**

(21) Anmeldenummer: **87106658.5**

(22) Anmeldetag: **07.05.87**

(51) Int. Cl.[5]: **A23K  3/02**, C12N 1/20,
//(C12N1/20,C12R1:25)

(54) **Verfahren zum Einsäuern von Grünfutter.**

(30) Priorität: **14.05.86 DE 3616181**

(43) Veröffentlichungstag der Anmeldung:
**07.01.88 Patentblatt  88/01**

(45) Bekanntmachung des Hinweises auf die
Patenterteilung:
**30.09.92 Patentblatt  92/40**

(84) Benannte Vertragsstaaten:
**AT BE CH DE FR GB IT LI NL SE**

(56) Entgegenhaltungen:
**AT-A- 262 040**
**DE-A- 2 736 880**
**FR-A- 2 542 013**
**US-A- 4 528 199**

(73) Patentinhaber: **SANOFI-CEVA Gesellschaft mit
beschränkter Haftung
Kanzlerstrasse 6
W-4000 Düsseldorf(DE)**

(72) Erfinder: **Beck, Theodor, Dr.
Belgradstrasse 142
W-8000 München 40(DE)**
Erfinder: **Gross, Friedrich, Dr.
Prof.-Zorn-Strasse 8
W-8011 Grub(DE)**

(74) Vertreter: **Türk, Gille, Hrabal, Leifert
Brucknerstrasse 20
W-4000 Düsseldorf 13(DE)**

EP 0 250 786 B1

## Beschreibung

Die Erfindung betrifft ein Verfahren zum Einsäuern von Grünfutter unter Verwendung von Bakterienpräparaten aus Milchsäure erzeugenden Bakterien.

Das Einsilieren von Grünfutter hat außerordentliche wirtschaftliche Bedeutung, und es sind verschiedene Verfahren bzw. Zusatzstoffe zur Beschleunigung der Vergärung von Grünfuttermassen bzw. zur Verbesserung der Silagen vorgeschlagen worden, einschließlich des Zusatzes von Milchsäure erzeugenden Bakterien, da die Milchsäuregärung ein wesentlicher Vorgang beim Einsilieren von Grünfutter ist.

Eine gute Silage sollte am Ende der Gärung etwa 2 bis 3 Gew.-% Milchsäure enthalten. Die zugesetzten Bakterien sollten alle oder möglichst viele der im Grünfutter enthaltenen Zucker vergären können. Diese Aufgabenstellung wird aber von den bekannten Bakterien, die man bisher den Silagen zugesetzt hat, nicht ausreichend gelöst (vergl. OE-A 262 040).

Der vorliegenden Erfindung liegt deshalb die Aufgabe zugrunde, Bakterienpräparate bzw. Bakterien zur Verfügung zu stellen, die verbesserte Silagen liefern, insbesondere in schnellerer Zeit eine stärkere Säuerung der Silage bewirken, wobei eine Buttersäuregärung weitgehend vermieden wird.

Gegenstand der Erfindung ist demgemäß ein Verfahren zum Einsäuern von Grünfutter unter Verwendung von Bakterienpräparaten aus Milchsäure erzeugenden Bakterien, das dadurch gekennzeichnet ist, daß man Lactobacillus plantarum DSM 3676 und/oder Lactobacillus plantarum DSM 3677 einsetzt.

Es wurde überraschenderweise gefunden, daß die beiden Mikroorganismen DSM 3676 und DSM 3677, wenn sie gemeinsam eingesetzt werden, eine ergänzende Wirkung haben. Bevorzugt ist deshalb, daß ein Gemisch aus, bezogen auf die Anzahl der Keime, 10 bis 90 % Lactobacillus plantarum DSM 3676 und 90 bis 10 % Lactobacillus plantarum DSM 3677, eingesetzt wird. Besonders bevorzugt ist ein Gemisch von 30 bis 70 % DSM 3676 und 70 bis 30 % DSM 3677, insbesondere 40 bis 60 % DSM 3676 und 60 bis 40 % DSM 3677.

Die Erfindung betrifft auch die Verwendung von Lactobacillus plantarum DSM 3676 und/oder Lactobacillus plantarum DSM 3677 zum Einsilieren von Grünfutter.

Die beiden obengenannten gemäß der Erfindung eingesetzten Mikroorganismen sind bisher nicht beschrieben. Der eine dieser Mikroorganismen wurde von der Anmelderin zunächst mit der Bezeichnung B 5 versehen und am 12. März 1986 bei der Deutsche Sammlung von Mikroorganismen, Grisebachstr. 8, D-3400 Göttingen, hinterlegt und erhielt die Eingangsnummer DSM 3676. (Hinterlegung nach dem Budapester Vertrag über die internationale Anerkennung der Hinterlegung von Mikroorganismen für die Zwecke von Patentverfahren.)

Der andere Mikroorganismus wurde von der Anmelderin zunächst mit der Bezeichnung W 2 versehen und ebenfalls am 12. März 1986 bei der gleichen Hinterlegungsstelle hinterlegt. Ihm wurde die Eingangsnummer DSM 3677 zugeteilt.

Beide Mikroorganismen wurden als Lactobacillus plantarum, Subspecies arabinosus identifiziert. Sie zeigen folgende Eigenschaften:

keine Gasbildung

DL-Milchsäure

kein Ammoniak aus Arginin

Temperaturoptimum ca. 35°C

kein Wachstum bei 45°C

Wachstum bei 15°C

Zellwandbestandteil:

Diaminopimelinsäure

Zuckervergärung

Glucose                    positiv

Mannit                     positiv

Glyzerin                   negativ

Arabinose                  positiv

Saccharose                 positiv

Lactose                    positiv

Melibiose                  positiv

Dextrin                    positiv (bei W 2)
                                   (bei B 5 schwächer)

Stärke                     negativ

Esculin                    positiv

Maltose                    positiv

Melizitose                 positiv

Xylose                     negativ

Ripose                     positiv

Trihalose                  positiv

Rafinose                   positiv

Sorbit                     positiv

Salizin                    positiv

Rhamnose                   positiv (bei W 2)
                                   (bei B 5 negativ)

Unterschiede:

W 2 bildet Schleim

B 5 bildet keinen Schleim

Azetatverträglichkeit:

für W 2 ist 1,5 %

für B 5 ist 0,5 %

Morphologie:

W 2 = Stäbchen einzeln

B 5 = Kurzstäbchen in Ketten


Säurung in MRS

beim B 5 3,81 pH

beim W 2 3,79 pH


Stoffwechsel für B 5 bestimmt: Aus einem Mol Glucose werden 2 Mol Lactat und aus einem Mol Dripose werden 1 Mol Lactat + 0,65 Mol Azetat und Ethanol in Spuren gebildet.

Die Mikroorganismen können in üblicher Weise gezüchtet werden. Geeignete Züchtungsbedingungen in MRS-Medium:

pH vor Sterilisation: 6.9

Sterilisation 20 min bei 121°C

pH nach Sterilisation: 6.6

Verhalten gegenüber Sauerstoff:

mikroaerophil (+)

Auf Konservierungsagar bei Kühlschrankaufbewahrung.

Bebrütungstemperatur: 25 bis 32°C

Bebrütungsdauer: 24 Std.

Überimpfintervall: etwa 3 Monate

Geeignete Aufbewahrungsbedingungen:

a) Stichkultur in Konservierungsagar (wie für Lactobacillen üblich). Nach dem Anwachsen, im Kühlschrank aufbewahren.

b) Gefriergetrocknete Kultur bei -20°C nahezu unbegrenzt lagerfähig.

Bedingungen für die Lebensfähigkeitsprüfung:

Plattenguß oder Stichkultur in MRS-Medien.

Die neuen Mikroorganismen zeichnen sich durch besondere Fähigkeiten in der Intensität und Geschwindigkeit der Fermentierung von Zuckern, wie sie in Grünfutter vorliegen, bei den üblichen Silagetemperaturen aus.

Die Mikroorganismen wurden aus Grünfuttersilagen in früher Gärphase isoliert.

In den nachfolgenden Tabellen sind die Ergebnisse beschrieben, die beim Einsäuern von Gras bzw. Luzerne unter Verwendung der Mikroorganismen gemäß der Erfindung sowie Siliermitteln des Stands der Technik erzielt wurden. Je niedriger der pH-Wert ist, desto stärker ist die erzielte Säuerung und umso besser ist dies für die Silage.

In den Tabellen wurden folgende Abkürzungen verwendet:

TS: Trockensubstanz

Kofa-Lac (eingetragenes Warenzeichen): Handelspräparat gemäß Stand der Technik

Kofa-Plus: Handelspräparat gemäß dem Stand der Technik auf Basis von Chemikalien

Die Zahlen $10^5$ bzw. $10^6$ geben die Anzahl der Keime pro 1 ml bzw. 1 g Frischmasse an (Impfstärke).

Unster.: Unsteril

Biomax (eingetragenes Warenzeichen): Handelspräparat gemäß dem Stand der Technik

Die in den Tabellen angegebenen Zahlen sind die jeweiligen pH-Werte.

Tab. 1 Säuerungsgeschwindigkeit (pH-Absenkung) in sterilen und unsterilen, künstlichen (MRS-A bis C je nach Impfstärke) und natürlichen Medien (2 Ansätze) bei 20°C und 30°C. Impfstärke pro 10 ml: $5 \times 10^5 - 5 \times 10^6$ Keime

| Versuch Nr. | | 1 | 2 | 3 | 4 | 5 | 6 | 7 | 8 | 9 | Mittel aus Versuch. | | |
|---|---|---|---|---|---|---|---|---|---|---|---|---|---|
| Nährmedium | | Gras + Luzerne unster. | Mais A unster. | MRS/A steril | MRS/B steril | Gras + Luzerne unster. | Mais + Luzerne steril | Mais B unster. | Mais B steril | MRS/C steril | 1 - 9 | 5 - 9 | 1 - 3 |
| pH steril, 0 | | 6.28 | 5.78 | 6.49 | 6.48 | 6.34 | 6.41 | 5.81 | 6.02 | 6.49 | | | |
| 20 °C/24 h | Kofa-Lac | 5.22 | 5.43 | 5.65 | 6.00 | 6.00 | 5.80 | 5.48 | 5.41 | 6.33 | 5.70 | 5.80 | 5.43 |
| | Biomax | 5.87 | 5.52 | 6.26 | | | | | | | | | 5.88 |
| | W 2 | 4.98 | 4.37 | 5.31 | 5.82 | 5.39 | 5.70 | 4.67 | 5.22 | 6.00 | 5.27 | 5.40 | 4.98 |
| | B 5 | 4.51 | 4.23 | 4.75 | 4.97 | 4.95 | 5.05 | 4.54 | 5.21 | 4.95 | 4.80 | 4.94 | 4.50 |
| 30 °C/24 h | Kofa-Lac | 4.31 | 3.51 | 4.02 | 4.00 | | 4.28 | | | 3.98 | 4.02 | 3.95 | 4.09 |
| | Biomax | 4.38 | 3.59 | 4.11 | | | | | | | | 4.03 | |
| | W 2 | 4.16 | 3.47 | 3.97 | 3.99 | | 4.22 | | | 3.99 | 3.97 | 3.87 | 4.07 |
| | B 5 | 4.33 | 3.88 | 4.19 | 4.18 | | 4.33 | | | 4.18 | 4.18 | 4.13 | 4.23 |

EP 0 250 786 B1

Tabelle 2

Gras TS ca- 18 % Ts, Weckglas $22^{o}$C und $30^{o}$C     pH-Entwicklung in Tagen

| Variaten | | $30^{o}$C | | | | $22^{o}$C | |
|---|---|---|---|---|---|---|---|
| | 0 | 1 1/2 | 4 | 14 | | 4 | 14 |
| 0 | | 5.21 | 4.72 | 5.41 | | 4.57 | |
| (Kofa-Lac | | 4.64 | 4.46 | 4.48 | | 4.46 | |
| $10^{6}$ ($B_5$ | 5.68 | 4.54 | * | * | | 4.43 | ** |
| ($W_2$ | | 4.53 | 4.41 | 4.48 | | 4.44 | |
| (Kofa-Lac | | 4.73 | 4.62 | 4.76 | | | |
| $10^{5}$ ($B_5$ | | 4.69 | 4.50 | * | | | |
| ($W_2$ | | 4.56 | 4.51 | * | | | |
| Kofa-Plus | | 4.84 | 4.45 | 4.35 | | | |

*   noch nicht geöffnet

**  Gärgasverluste 1.65-2.25 g /Gefäß
    keine gesicherten Unterschiede in den Impfvarianten

EP 0 250 786 B1

Tabelle 3

Säuerung im Preßsaft (pH direkt gemessen)

Gras TS ca. 18 %

| | Preßsaft | | | | | |
| --- | --- | --- | --- | --- | --- | --- |
| | 30°C | | | 22°C | | |
| | 21h | 45h | 4Tg | 21h | 45h | 4Tg |
| 0 | 4.71 | 4.04 | 4.05 | 5.67 | 4.44 | 4.13 |
| (Kofa-Lac | 4.07 | 3.95 | 3.93 | 5.65 | 4.20 | 4.12 |
| $10^6$(B$_5$ | 3.95 | 3.95 | 3.93 | 4.84 | 4.15 | 4.10 |
| (W$_2$ | 4.02 | 3.97 | 3.97 | 4.95 | 4.12 | 4.09 |
| (Kofa-Lac | 4.28 | 4.00 | 3.98 | | | |
| $10^5$(B$_5$ | 4.02 | 3.97 | 3.93 | | | |
| (W$_2$ | 4.03 | 4.00 | 3.98 | | | |

EP 0 250 786 B1

**Tabelle 4**

**Säuerung im Preßsaft**

Preßsaft — Luzerne frisch

| | 22°C | | | 30°C | | |
|---|---|---|---|---|---|---|
| | 1Tg | 2Tg | 4Tg | 1Tg | 2Tg | 4Tg |
| 0 | 5.95 | 5.15 | 5.03 | 5.18 | 4.76 | 4.76 |
| (Kofa-Lac 10⁶ (B5 | 5.91 | 4.80 | 4.79 | 4.46 | 4.44 | 4.43 |
| (W₂ | 5.61 | 4.55 | 4.53 | 4.35 | 4.30 | 4.30 |
| (RP | 5.69 | 4.60 | 4.60 | 4.41 | 4.36 | 4.35 |
| | | | | 4.57 | 4.41 | 4.40 |
| (Kofa-Lac 10⁵ (B5 | | | | 4.70 | 4.61 | 4.66 |
| (W₂ | | | | 4.52 | 4.48 | 4.47 |
| | | | | 4.50 | 4.48 | 4.47 |

Die Züchtung der Bakterien kann in an sich bekannter Weise erfolgen (vergl. OE-A- 262 040). Während der Züchtung kann gegebenenfalls eine alkalisch wirkende Substanz dem Nährmedium zugesetzt werden, um den pH-Wert nicht zu stark absinken zu lassen. Beispiele für solche alkalische Substanzen sind Kaliumhydroxidlösung, Natriumhydroxidlösung, Sodalösung. Das Abtrennen der Zellen kann in bekannter Weise erfolgen. Beispielsweise können die Zellen in einer Zentrifuge abgetrennt werden. Man kann ihnen anschließend einen Schutzstoff, beispielsweise ein Schutzkolloid, z.B. Milchpulver, zusetzen. Es können auch andere Schutzstoffe, beispielsweise Lactose, Ascorbinsäure, verwendet werden.

Die Zellen können dann gefriergetrocknet werden. Man erhält ein stabiles Präparat, das die aktiven Bakterien enthält und lange Zeit ohne Verlust ihrer Aktivität haltbar ist.

Das Präparat kann in bekannter Weise mit Hand, Dosiergeräten, Pulverspritzen und dergleichen dem einzusilierenden Gut zugesetzt werden.

US-A-4 528 199 und AT-A-262 040 beschreiben die Verwendung von Lactobacillus plantarum Präparaten zum Einsäuern von Grünfutter. Gegenüber den darin verwendeten Präparaten zeigen die erfindungsgemäß verwendeten Bakterien überraschenderweise verbesserte Eigenschaften hinsichtlich der Säuerungsgeschwindigkeit.

**Patentansprüche**

1. Verfahren zum Einsäuern von Grünfutter unter Verwendung von Bakterienpräparaten aus Milchsäure erzeugenden Bakterien, **dadurch gekennzeichnet,** daß man Lactobacillus plantarum DSM 3676 und/oder Lactobacillus plantarum DSM 3677 einsetzt.

2. Verfahren nach Anspruch 1, dadurch gekennzeichnet, daß man ein Gemisch aus, bezogen auf die Anzahl der Keime, 10 bis 90 % Lactobacillus plantarum DSM 3676 und 90 bis 10 % Lactobacillus plantarum DSM 3677 einsetzt.

3. Verfahren nach Anspruch 1, dadurch gekennzeichnet, daß man ein Gemisch aus, bezogen auf die Anzahl der Keime, 30 bis 70 % Lactobacillus plantarum DSM 3676 und 70 bis 30 % Lactobacillus plantarum DSM 3677 einsetzt.

4. Verwendung von Lactobacillus plantarum DSM 3676 und/oder Lactobacillus plantarum DSM 3677 zum Einsilieren von Grünfutter.

5. Biologisch reine Kultur von Lactobacillus plantarum DSM 3676.

6. Biologisch reine Kultur von Lactobacillus plantarum DSM 3677.

**Claims**

1. A method of ensilage of green fodder using preparations of lactic acid-producing bacteria, characterised in that Lactobacillus plantarum DSM 3676 and/or Lactobacillus plantarum DSM 3677 is used.

2. A method according to claim 1, characterised in that a mixture of 10 to 90% Lactobacillus plantarum DSM 3676 and 90 to 10% Lactobacillus plantarum DSM 3677 is used, relative to the number of germs.

3. A method according to claim 1, characterised in that a mixture of 30 to 70% Lactobacillus plantarum DSM 3676 and 70 to 30% Lactobacillus plantarum DSM 3677 is used relative to the number of germs.

4. Use of Lactobacillus plantarum DSM 3676 and/or Lactobacillus plantarum DSM 3677 for ensilage of green fodder.

5. A biologically pure culture of Lactobacillus plantarum DSM 3676.

6. A biologically pure culture of Lactobacillus plantarum DSM 3677.

**Revendications**

1. Procédé d'acidification de fourrage vert par utilisation de préparations bactériennes provenant de bactéries productrices d'acide lactique, caractérisé en ce qu'on utilise Lactobacillus plantarum DSM 3676 et/ou Lactobacillus plantarum DSM 3677.

2. Procédé selon la revendication 1, caractérisé en ce qu'on utilise un mélange oonstitué, par rapport au nombre des germes, de 10 à 90% de Lactobacillus plantarum DSM 3676 et de 90 à 10% de Lactobacillus plantarum DSM 3677.

3. Procédé selon la revendication 1, caractérisé en ce qu'on utilise un mélange constitué, par rapport au nombre des germes, de 30 à 70% de Lactobacillus plantarum DSM 3676 et de 70 à 30% de Lactobacillus plantarum DSM 3677.

4. Utilisation de Lactobacillus plantarum DSM 3676 et/ou de Lactobacillus plantarum DSM 3677 à l'ensilage de fourrage vert.

5. Culture biologiquement pure de Lactobacillus plantarum DSM 3676.

**6.** Culture biologiquement pure de Lactobacillus plantarum DSM 3677.